# EUROPÄISCHE PATENTSCHRIFT

(11) **EP 0 525 292 B1**
(45) Veröffentlichungstag und Bekanntmachung des Hinweises auf die Patenterteilung: **24.08.1994**
(21) Anmeldenummer: 92104086.1
(22) Anmeldetag: 10.03.1992
(51) Int. Cl.: A61K 7/06, A61H 33/02, A45D 19/00, A61K 7/50, B01F 5/04, A61K 7/00

(54) **Verfahren und Anordnung zur Behandlung von Haar, Kopfhaut und/oder Körperhaut**
Process and device for treating hair, scalp and/or skin
Procédé et appareil pour le traitement des cheveux, du cuir chevelu et/ou de la peau

(30) Priorität: 24.05.1991 DE 4117023; 25.07.1991 DE 4124728; 10.01.1992 DE 4200467
(43) Veröffentlichungstag der Anmeldung: 03.02.1993
(73) Patentinhaber: Technica Entwicklungsgesellschaft mbH & Co. KG, 23909 Ratzeburg (DE)
(72) Erfinder: Kückens, Alexander, W-2401 Gross-Sarau (DE); Köhl, Horst, W-2060 Bad Oldesloe (DE)
(74) Vertreter: Fricke, Joachim, Dr.

(56) Entgegenhaltungen:
- EP-A- 0 155 683
- EP-A- 0 170 269
- EP-A- 0 176 694
- DE-A- 1 491 563
- DE-A- 3 610 266
- DE-A- 3 618 726
- DE-A- 3 840 567

## Beschreibung

Es ist bekannt, daß Haare ebenso wie Kopf- und Körperhaut durch Umwelteinflüsse ebenso wie durch die für die reinigende, kosmetische, therapeutische oder Kurzwecken dienende Behandlung verwendeten Mittel und Methoden starken Belastungen unterworfen sind. Dabei spielt eine Rolle, daß bei bestimmten Behandlungen ein Quellen der Zellen oder Poren von Haut oder Haar bzw. ein Spreizen der Haarschuppen hilfreich oder sogar notwendig sind. Dabei öffnen sich die Schuppen und Poren und können somit den wässrigen Behandlungsstoffen einen besseren Zugang und eine bessere Einwirkungsmöglichkeit geben. Solche Quellvorgänge können z.B. durch alkalisch eingestellte wässrige Behandlungsstoffe begünstigt werden, im sauren Bereich liegende Mittel führen dagegen zu einer Umkehrung dieses Vorganges, d.h. diese wirken häufig glättend und zusammenziehend. So sind z.B. Haarglänz-Spülmittel säuernd eingestellt, so daß sich die Schuppen anlegen und so zusätzlich zu der Verwendung von Fett- und Glanzstoffen auch eine natürliche Glanzwirkung durch eine gleichmäßiger anliegende Schuppenschicht mit zur Wirkung gebracht werden kann. Zur Ansäuerung von Haarglanzmitteln sind verschiedene schwache Säuren, wie Zitronensäure Weinsäure und dgl. bekannt. Für die Erhaltung oder Wiederherstellung des Säureschutzmantels der Haut werden ebenfalls Puffergemische der verschiedensten Art verwendet, z.B. Ammoniummonohydrogenzitrat in Verbindung wasserfreier Zitronensäure in einer Öl-in-Wasser-Emulsion oder dergleichen.

All diese Stoffe sind aber, insb. bei Vorliegen von Hauterkrankungen, offenen Wunden oder besonders empfindlichen Hautbereichen nicht, oder nur nach ärztlicher Konsultation einsetzbar.

Auf der anderen Seite sind zur Pflege oder zur Therapie dienende Voll-, Teil- oder Brausebäder bekannt, die neben den verschiedensten Säuren und Spurenelementen auch Kohlendioxyd und Kohlensäure enthalten. Es wird hierzu beispielsweise auf die verschiedenartig zusammengesetzten natürlichen Quellwässer von Heilbädern verwiesen. Man weiß heute, daß der eigentliche therapeutische Effekt bei diesen Heilquellen der Gehalt an CO₂ bzw. H₂CO₃ ist und daß die weiteren, von Quelle zu Quelle verschiedenen Bestandteile ohne gravierenden Einfluß sind.

Es sind ferner für die häusliche Anwendung Badezusätze bekannt, die zumeist aus mehreren Komponenten, darunter Säureträger und Säurebildner, zusammengesetzt sind, welche beim Einbringen in das Badewasser unter anderem CO₂-Gas aufperlen lassen (vgl. hierzu beispielsweise US-PS 4 666 707 oder DE-OS 36 18 726). Oder man hat Wasser unter hohem Druck mit CO₂-Gas imprägniert und für den Verkauf in Druckflaschen abgefüllt, um es dann zur Verwendung im Badewasser zu verteilen.

Die verschiedenen zusammengesetzten Badezusätze enthalten ebenfalls Säuren, wie Zitronensäure oder dergleichen und entwickeln mit den anderen Stoffen oft nur schwer überschaubare synergetische Wirkungen.

Die bekannten Maßnahmen sind auch relativ aufwendig. Hinzu kommt, daß es häufig üblich oder notwendig ist, nach einer entsprechenden Behandlung noch warm oder kalt nachzuspülen oder nachzubrausen. Bei vielen therapeutischen, kosmetischen, reinigenden oder pflegenden Zusatzmitteln, insb. solchen mit nur begrenzter Einwirkdauer, ist ein solche Nachspül- oder Nachbrausevorgang oder dgl. unbedingt erforderlich und vorgeschrieben.

Der Erfindung liegt die Aufgabe zugrunde, auf einfache, kostensparende Weise einer behandelnden Person, auch Selbstbehandlern, die Möglichkeit zu geben, die Gesundung zu fördern und natürliche Widerstandskräfte von Haar, Kopf- und Körperhaut zu stärken.

Diese Aufgabe wird durch die Lehre jeweils der Ansprüche 1, 2, 3 sowie 15 gelöst.

Die Erfindung geht von den in jedem Behandlungsraum, wie therapeutischen Bädern, Friseur- und Kosmetikgeschäften, oder aber auch in jedem Bad von Wohnungen üblicherweise zur Verfügung stehenden Druckwasserquellen, wie der übliche Wasseranschluß an das Versorgungsnetz, Warmwasserdruckspeicher oder Durchlauferhitzer oder dgl. aus. Das so zur Verfügung stehende Waser ist in der Regel aus Korrosionsschutzgründen so aufbereitet, daß sein pH-Wert im alkalischen Bereich liegt. Jedes Bad, jedes Nachspülen oder Nachbrausen oder dgl. mit diesem Wasser läßt somit das Haar, die Kopfhaut, die Schuppen. oder die Hautzellen aufquellen und im aufgequellten Zustand zurück, und zwar auch bei Verwendung von kaltem Wasser. Im aufgequollenen oder gespreizten Zustand halten die Zellen und Poren im verstärkten Maße Reste von Schmutzteilen und Behandlungsmitteln zurück, die bei der nachfolgend eintretenden Entquellung unter den Schuppen und in den Poren eingeschlossen bleiben. Außerdem bieten die gequollenen Zellen oder abgespreizten Schuppen oder dgl. schädigenden Stoffen oder Erregern verstärkte Eindringmöglichkeit.

Hier greift die Lehre nach der Erfindung entscheidend ein, indem sie auf sehr einfache Weise und an jeder Behandlungsstelle, insb. auch zu Hause, die Gewähr gibt, daß mit wenigstens dem letzten Nachspülen, Nachbrausen oder dgl. alle Zellen, Schuppen oder Poren von Haut und/oder Haar mit solchem Wasser in Kontakt gebracht werden, welches auf einen vorbestimmten abgesenkten pH-Wert angesäuert ist und adstringierende Wirkung entfaltet.

Wesentlich ist dabei, daß die sonst üblichen synergetischen Wirkungen ganz vermieden werden. Dem hier verwendeten Wasser werden also keine Stoffkompositionen zugesetzt. Vielmehr wird das Wasser - abgesehen von den Maßnahmen nach den Ansprüchen 6 und 7 - allein mit CO₂ versetzt, das unter Bildung von H₂CO₃ im Wasser außerordentlich stabil physikalisch gebunden wird.

Wesentlich ist ferner, daß zur pH-Wertabsenkung und Ansäuerung des Wassers ausschließlich das CO₂ mit der daraus gebildeten Kohlensäure eingesetzt wird. CO₂ und H₂CO₃ stehen im Gegensatz zu allen anderen Säurebildnern in einem biologisch natürlichen Verhältnis zu den Haar- und Körperzellen und nur diese Stoffe können im Inneren der Zellen pH-Wertabsenkungen im merklichen Umfange hervorrufen, und zwar schon bei sehr kurzer Einwirkzeit von 1 bis 2 Minuten. Dadurch wird das Herauslösen oder Ausscheiden von hautfremden Anteilen, wie Schmutzteilchen oder Resten der Behandlungsstoffe einer vorangegangenen Behandlung begünstigt. Die Zellen werden entquellt und adstringiert. Haar und Haut erfahren dadurch eine natürliche Glättung. Körpereigene, z.B. wichtige wasserbindende Substanzen, die im alkalischen Bereich aus den Poren und Zellen herausgelöst werden, bleiben bei der Nachbehandlung gemäß der Erfindung der Haut erhalten. Hinzu kommt die bakteriozide und bakteriostatische Wirkung der Kohlensäure, welche durch die Maßnahmen der Ansprüche 6 und 7 noch verstärkt werden kann. Die Einwirkung des hautfreundlichen CO₂ fördert eine starke Durchblutung der Kopfhaut und der Körperhaut. Die Nachbehanldung ist selbst bei geschädigter Haut, z.B. Hauterkrankungen oder dgl., oder bei offenen Wunden nicht nur unschädlich, sondern vielmehr von förderndem Einfluß auf den Heilungsprozeß. Dies gilt für relativ verschiedene Arten von Hauterkrankungen oder Schädigungen. Alles in allem werden Gesundung und Widerstandskraft von Haar und Haut wesentlich gefördert.

Wichtig für den Erfolg der neuen Lehre ist auch, daß die Anwendung nach jeder beliebigen Vor- oder Hauptbehandlung, wie Waschen, Färben, Formlegen, Einlegen mit Kurmitteln bei Haaren und Kopfhaut, oder wie Reinigen, kosmetisches oder therapeutisches Baden bezüglich sonstiger Körperpartien, im Rahmen des gewohnten Nachspül- oder Nachbrause- oder dgl. Nachbehandlungsvorganges erfolgen kann.

Wichtig ist ferner, daß unabhängig von der Härte oder dem Ausgangs-pH-Wert des Wassers und unabhängig von Druck- oder Strömungsschwankungen ein auf einen optimalen Wert abgesenkter pH-Wert voreingestellt und danach automatisch genau und zuverlässig aufrechterhalten werden kann, ohne daß die behandelnde oder die behandelte Person aufpassen oder eingreifen muß. Mit Aufdrehen des Wasserhahnes setzt vielmehr unmittelbar die Nachbehandlungswirkung mit dem voreingestellten pH-Wert voll ein.

Voraussetzung dafür sind die sehr genaue stabile CO₂-Imprägnierung des Wassers und die genaue und voll automatische CO₂-Gasdosierung. Das ganze ist mit einer Anordnung ermöglicht, die sich leicht an jeder üblichen Behandlungs- oder Bade- oder Brause- oder Waschstelle mit der dort üblichen und vorhandenen Armatur anbringen läßt. Die angestrebten Funktionen sind somit voll in den normalen Ablauf eines Dusch- oder Wasch- oder sonstigen Behandlungsvorganges integriert.

Durch einmalige Voreinstellung eines Grundwertes für den CO₂-Gasanteil kann der jeweils zur Verfügung stehenden Wasserqualität (z.B. pH-Wert, Karbonatanteile usw.) Rechnung getragen werden. Spätere Nachregelungen sind zumeist nicht erforderlich.

Die optimalen pH-Werte für die Haarbehanldung liegen dabei zwischen 5 und 6, insb. um 5,2 bis 5,5, und für die Behandlung der Körperhaut etwa zwischen 6 und 6,7.

Die Erindung ist auch da anwendbar, wo bei einer Vorbehandlung oder Hauptbehandlung in der bekannten Weise bereits CO₂ als einer von mehreren Wirkstoffen dem Wasser zugesetzt worden ist. Während in diesem Zusammenhang das CO₂ mit den anderen Stoffen synergetische Effekte erzeugt, entfaltet das CO₂ und die damit gebildete Kohlensäure allein ihre die Gesundung und Widerstandskraft von Poren, Schuppen, Hautteilchen oder dgl. fördernde Wirkung.

Wenn ätherische Öle oder Duftstoffe und /oder Silikonöl verwendet werden, kann der Kontakt des CO₂-Gases mit diesen Stoffen an jeder geeigneten Stelle des Weges des CO₂-Gases von der Druckgaspatrone zu dem beschleunigt fließenden Wasser erfolgen. Bevorzugt werden diese Stoffe jedoch bereits fabrikmäßig tröpfchenweise in den Druckgasbehälter oder die Druckgaspatrone während oder vor dem Einfüllen des CO₂-Gases eingebracht. Die Verteilung und Mischung dieser Stoffe mit dem Gas wird durch die Gasentspannung während der Entnahme des CO₂-Gases begünstigt.

Das plötzliche Ausbleiben des Duftes kann in diesem Falle mit Vorteil als Anzeige dafür ausgenutzt werden, daß der Druckgasbehälter entleert ist.

Die ätherischen Öle oder Duftstoffe werden zweckmäßigerweise so ausgewählt, daß sie, insb. aufgrund von Bestandteilen, wie Aldehyd und Ketonen, auf Mikroorganismen stark giftig wirken. Zu diesen Ölen gehören unter anderen Thymianöl, Eukalyptusöl, Kümmelöl oder dgl. , welche alle bakterizide Wirkungen besitzen. Diese Öle entfalten somit neben der Glanz- und Duftwirkung auch noch Wirkungen, welche zur Gesundheit von Haar und Haut wesentlich beitragen.

Bekanntlich dienen Silikone unter anderem auch als Bestandteile oder Grundlagen von Salben und wirken stabilisierend auf das Haar und sorgen vor allem für eine gute Haftung der ätherischen Öle auf dem Haar und der Haut. Die vorgeschlagenen Öle und ätherischen Stoffe üben außerdem eine milde Reizung auf die Haut aus und tragen so zur Heilwirkung bei. Bevorzugt wird eine Mischung aus ätherischen Ölen und Silikonölen.

Die Erfindung wird nachfolgend anhand schematischer Zeichnungen an mehreren Ausführungsbeispielen näher erläutert.

Es zeigen:
- Figur 1: in vereinfchter Darstellung und in Seitenansicht eine erste Anordnung zum Ausführen der neuen Verfahren;
- Figur 2: in gleicher Darstellung eine weitergebildete Ausführung der Anordnung zur Ausführung des Verfahrens nach Anspruch 6;
- Figur 3: im größeren Maßstabe und teilweise geschnitten die Anordnung, mit deren Hilfe das Wasser automatisch mit dem gewünschten Gehalt an H₂CO₃ angereichert werden kann, bevor er den Brausekopf oder eine andere Entnahmestelle erreicht;
In den Figuren sind für gleiche Teile gleiche Bezugszeichen verwendet.

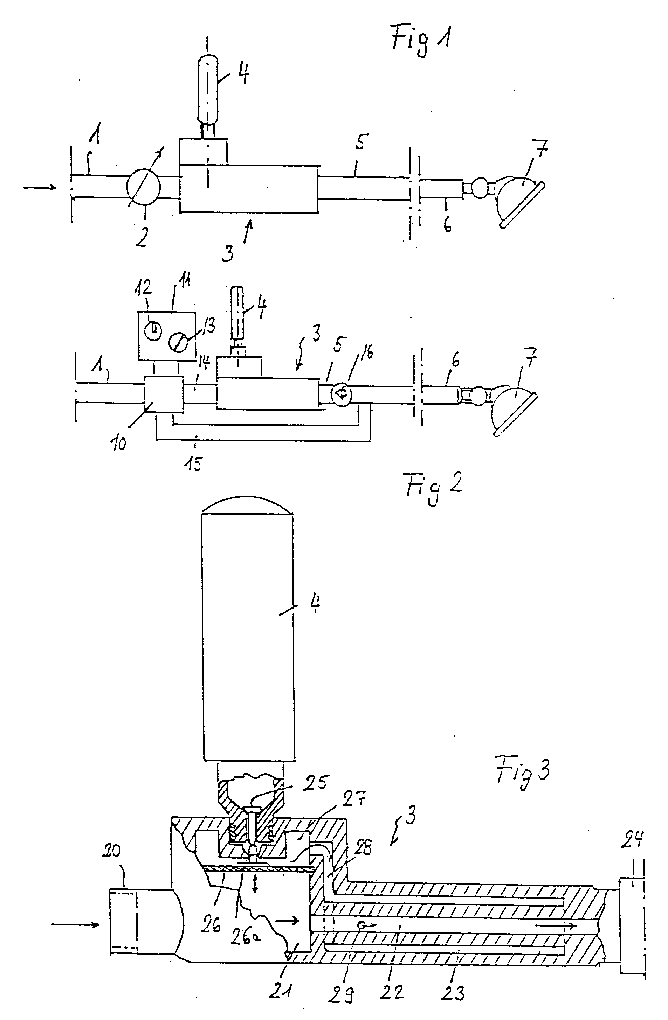

Figur 1 zeigt eine Leitung 1 von vorbestimmtem Einströmquerschnitt, die mit einer Druckwasserquelle, z.B. einer Wasserleitung, einem Durchlauferhitzer, einem Warmwasserspeicher oder dgl. verbunden werden kann. Bevorzugt wird das dem Einströmquerschnitt zugeführte Wasser auf eine Temperatur zwischen 25°C und 40°C erwärmt.

An diese Leitung ist über ein Ein/Aus-Schaltventil eine Vorrichtung 3 angeschlossen, die in Figur 3 näher dargestellt ist.

Gemäß Figur 3 besteht die Vorrichtung aus einem Block, in dem an einem Ende eine Wasser-Vorkammer 21 vorgesehen ist. Diese ist über einen Anschluß 20 von vorbestimmtem Einströmquerschnitt an das Ventil 2 anschließbar. Von der Wasservorkammer 21 geht ein Ausströmkanal 22 aus, dessen Querschnitt deutlich geringer, vorzugsweise etwa nur halb so groß wie der Einströmquerschnitt ist. An seinem Ausströmende mündet der Ausströmkanal 22 in ein Anschlußstück 24, das mit einer Wasserentnahmestelle, z.B. mit einer flexiblen Leitung 5 nach Figur 1 verbunden werden kann, an derem Ende eine fest installierte Brause oder eine Handspülbrause 6 mit Brausekopf 7 angeschlossen ist. Die hinter dem Ausströmkanal 22 liegenden Strömungsquerschnitte sind so gewählt, daß sie keinenfalls kleiner als der Strömungsquerschnitt des Ausströmkanals 22 sind. Der Ausströmkanal 22 kann aber auch direkt als Zulauf in eine Liege- oder Sitzbadewanne münden.

Der Ausströmkanal 22 wird von einer Ringkammer umgeben, die einen Druckgasraum 23 bildet. Dieser Druckgasraum 23 steht über mindestens eine Bohrung 29 in direkter und freier Strömungsverbindung mit dem Ausströmkanal 22. Ferner ist der Druckgasraum 23 über einen Kanal 28 mit einer Vorkammer 27 verbunden. Die Gas-Vorkammer 27 steht mit der einen Seite und die Wasser-Vorkammer 21 mit der anderen Seite einer flexiblen Membran 26 in freier Strömungsverbindung. Die Membran 26 weist ein Betätigungsglied 26a auf, das durch eine Einströmöffnung des Gehäuseblockes ragt und auf das Schließglied 25 des automatisch und unter vorbestimmter Vorspannung schließenden Austrittsventils einer Gasdruckpatrone 4 oder dgl. Gasdruckquelle einwirkt. Bei der Gasdruckpatrone kann es sich bei einer Anordnung zum Haarspülen z.B. um eine Patrone mit 8 oder mehr Gramm CO₂-Gasinhalt handeln.

Das über den Einströmquerschnitt 20 zuströmende Wasser wirkt auf die Membran 26 im Öffnungssinne des Ventils 25. Das bei offenem Ventil ausströmende Gas entwickelt in der Vorkammer 27 einen Gasdruck, der dem auf der Membran 26 lastenden Wasserdruck entgegenwirkt. Es spielt sich so automatisch und unabhängig von der Strömungsgeschwindigkeit und dem Druck des Wassers ein vorbestimmtes Verhältnis zwischen Wasserdruck und Gasdruck in den beiden Vorkammern ein.

Das Wasser tritt aus der Wasser-Vorkammer 21 in den engeren Ausströmkanal 22 ein, wodurch sich die Strömungsgeschwindigkeit vergrößert, z.B. verdoppelt. Entsprechend senkt sich der statische Druck in dem Ausströmkanal ab, z.B. etwa auf die Hälfte des statischen Druckes des zuströmenden Wassers.

Die Anordnung ist so getroffen, daß sich in dem Druckgasraum 23 ein Gasdruck einspielt, der höher liegt als der Druck in dem Ausströmquerschnitt 22, jedoch niedriger als der Druck des Wassers im Zuströmquerschnitt. Dadurch wird sichergestellt, daß das Ventil 25 zuverlässig schließt, wenn der Wasserdruck in der Vorkammer 21 unter einen für den ordnungsgemäßen Betrieb notwendigen Druck absinkt.

In das durch den Ausströmkanal 22 mit höherer Geschwindigkeit strömende Wasser gelangt CO₂ aus dem Druckgasraum 23, und zwar in einem dem Druckverhältnis zwischen Wasser und Gas entsprechendem geringem Maße und so fein imprägniert, daß das CO₂-Gas über eine ausreichend lange Zeit gebunden bleibt.

Der Gasgehalt wird dem Anwendungsfall entsprechend eingestellt. Für das Haarspülen z.B. 400 mg CO₂ undmehr pro Liter Wasser, um in dem Wasser ausreichende Spurenmengen an H₂CO₃ für die Absenkung des pH-Wertes auf Werte deutlich unter 7 zu bilden. Da das Haarspülwasser unmittelbar nach Aufnahme der Spurenmengen an CO₂ von dem Spülkopf oder der Spülbrause 7 auf das Haar gelangt, besitzt das Haarspülwasser im Augenblick des Kontaktes mit dem Haar den gewünschten Gehalt an CO₂ und den gewünschten abgesenkten pH-Wert. Die Wirkung auf das Haar ist gleichbleibend, das das austretende Wasser nur kurzfristig mit dem Haar in Kontakt kommt und durch nachfließendes Wasser sofort ersetzt wird.

Da das Haar alle die Qualität des Haares beeinträchtigenden Stoffe an das mit Spuren an H₂CO₃ angereicherte Haarspülwasser abgibt, kann es zweckmäßig sein, diese Form der Haarspülung intervallmäßig abzuwechseln mit einer Haarspülung mit unbehandeltem Haarspülwasser, in dem kein Gehalt an H₂CO₃ gebildet ist. Dies kann durch Wechsel der Haarspülbrause erfolgen. Zweckmäßigerweise ist dafür jedoch die Anordnung nach Figur 2 vorgesehen. Bei dieser Anordnung ist zu der Vorrichtung 3 eine Umgehungsleitung 15 für das Haarspülwasser vorgesehen, die hinter der Vorrichtung 3 und hinter einem dieser Vorrichtung zugeordneten Rückschlagventil 16 in die zur Brause 7 führende Leitung mündet. Vor der Vorrichtung 3 ist ein Umschaltventil vorgesehen, welches abwechselnd die Vorrichtung 3 oder die Umgehungsleitung 15 mit der Zuführungsleitung 1 für das Haarspülwasser verbindet. Die Umsteuerung erfolgt beispielsweise durch eine Steuervorrichtung 11, an der durch entsprechende Einstellknöpfe 12 und 13 die Intervalldauer und die Intervallhäufigkeit eingestellt werden können. Der Ventilanordnung 10 kann noch ein Ein-Aus-Ventil vorgeschaltet sein. Diese Funktion kann aber automatisch auch durch die Ventileinrichtung 10 und die zugehörige Steuereinrichtung 11 übernommen werden.

Bei Anwendung für therapeutische Zwecke wird das für das Bad entsprechend erwärmte Wasser in der in den Figuren gezeigten Anordnung mit CO₂-Gas feinstimprägniert, so daß das Wasser das CO₂-Gas z.B. bei Voll- oder Sitzbädern nur langsam freigibt und über eine längere Badedauer von z.B. 20 bis 30 Minuten hinweg wirksam bleibt. Hierbei wird das Wasser in der Anordnung mit einem Gehalt an CO₂-Gas von mindestens etwa 350 mg/l Wasser, vorzugsweise jedoch 500 bis max. 1500 mg/l Wasser feinstimprägniert, d.h. im wesentlichen ohne größere, einem besonderen Auftrieb im Wasser unterliegenden Gasblasen. In vielen Fällen steigt die therapeutische Wirkung mit zunehmendem CO₂-Gehalt bis zum Erreichen von etwa 1250 bis 1400 mg/l Wasser an. Ein darüber hinaus gesteigerter CO₂-Gehalt bringt praktisch eine Wirkungszunahme nicht.

Das Wasser kann zweckmäßigerweise vor Einleiten in die Imprägnierungszone auf Temperaturen zwischen 25° C und 40° C erwärmt werden. Es ist aber auch möglich, das Wasser bei der üblichen Kaltwasser-Leitungstemperatur zu imprägnieren und nachträglich mit erhitztem Wasser auf die gewünschte Temperatur zu erwärmen.

In die Druckgaspatrone wird vor oder mit dem Füllen mit CO₂-Gas eine geringe Menge an ätherischem Duftstoff oder Öl oder Siliconöl oder eine Mischung daraus eingebracht.

Von besonderenn Vorteil ist, daß die Maßnahmen nach der Erfindung auch eine um 2-5° verringerte Bade- oder Brausetemperatur ermöglichen, ohne daß von der Person dies als kühler oder gar zu kühl empfunden wird.

Ein weiterer Vorteil der erfindungsgemäßen Maßnahmen liegt darin, daß die Lösung und Verteilung der geringen Mengen an ätherischen und/oder Silikonöl bei Einbringen schon in die Patrone durch das verflüssigte CO₂ außerordentlich begünstigt werden.

## Patentansprüche

1. Verfahren zur Haarpflege, bei dem
(a) das Haar nach einer Pflegebehandlung (Wasch-, Farb-, Umformungsbehandlung, Kurpackungsvorgang) einer Haarglanzspülung mit säuernder und adstringierender Wirkung unterworfen wird;
(b) zur Haarglanzspülung das einer Druckwasserquelle (Wasseerversorgungsleitung, Durchlauferhitzer, Warmwasserspeicher unmittelbar entnommene - ggf. warme - Wasser verwendet wird, dem als alleiniger Säurebildner reines CO₂-Gas aus einer CO₂-Druckgasquelle (4) mit einem Druck deutlich unterhalb des statischen Drucks des der Druckwasserquelle entnommenen Wassers an einer Stelle des Wasserströmungsweges (22,29) zugesetzt wird, an dem der statische Druck des Wassers unter den Druck des CO₂-Gases herabgesetzt wird;
(c) wobei der pH-Wert auf einen bestimmten Wert zwischen 5 und 6, insb. 5,2, genau und konstant geregelt wird.

2. Verfahren zur Herstellung einer Stoffmischung zur pflegenden, kosmetischen oder therapeutischen Behandlung der Haut, bei dem
einer Druckwasserquelle (Wasserversorgungsleitung, Durchlauferhitzer, Warmwasserspeicher) unmittelbar entnommenem - ggf. warmem - Wasser als alleiniger Säurebildner reines CO₂-Gas aus einer CO₂-Druckgasquelle (4) mit einem Druck deutlich unterhalb des statischen Druckes des der Druckwasserquelle entnommenen Wassers an einer Stelle des Wasserströmungsweges (22,29) zugeführt wird, an welcher der statische Druck des Wassers unter den Druck des CO₂-Gases herabgesetzt wird und der pH-Wert auf einen vorbestimmten Wert zwischen etwa 5 und 6,7 genau und konstant geregelt wird.

3. Verwendung von - ggf. erwärmtem - Wasser, das einer Druckwasserquelle entnommen und unmittelbar mit reinem CO₂-Gas als alleinigem Säurebildner aus einer Druckgasquelle (4) so versetzt ist, daß sich der pH-Wert des Wassers auf einen vorbestimmten Wert zwischen 6,7 und 5,2 konstant und genau einstellt, zur wenigstens letzten Nachbehandlung von Haar, Kopfhaut oder Körperhaut nach einer vorgeschalteten Pflegebehandlung.

4. Verfahren nach einem der Ansprüche 1 bis 3, **dadurch gekennzeichnet**,
daß das der Druckwasserquelle unter variierendem Druck zu einer Entnahmestelle (7,24) fließende Wasser dazu verwendet wird, den Druck des der Druckgasquelle (4) entnommenen CO₂ auf einem dem Wasserdruck entsprechenden, jedoch niedrigeren Druck selbsttätig einzuregeln, worauf der Druck des fließenden Wassers auf einen Wert unter den CO₂-Gasdruck absinkt und das CO₂ mit dem Wasser in direkten freien Austausch gebracht wird

5. Verfahren nach einem der Ansprüche 1 bis 4, **dadurch gekennzeichnet**,
daß man einen vorbestimmten festen Gasdruck-Grundwert in Abhängigkeit von der Beschaffenheit, insb. dem pH-Wert, des der Druckwasserquelle entnommenen Wassers grob voreinsteuert, insb. durch einmaliges Verstellen einer einstellbaren Nadeldüse im Zuströmweg (28) des CO₂-Gases.

6. Verfahren nach einem der Ansprüche 1 bis 5, **dadurch gekennzeichnet**,
daß man das CO₂-Gas vor seinem Kontakt mit dem fließenden Wasser mit einem ätherischen Öl oder Duftstoff in Kontakt bringt.

7. Verfahren nach Anspruch 6, **dadurch gekennzeichnet**,
daß man das Öl oder die Mischung aus Öl und Duftstoff tröpfchenweise mit dem CO₂-Gas bereits in einem - die Druckgasquelle bildenden - Druckgasbehälter (4) in Kontakt bringt.

8. Verfahren nach einem der Ansprüche 6 bis 7, **dadurch gekennzeichnet**,
daß man bei Betrieb den Abbruch der Wahrnehmbarkeit des ätherischen Öles oder Duftstoffes zugleich als Anzeige für eine Entleerung eines - die Druckgasquelle bildenden - Druckgasbehälters (4) heranzieht.

9. Verfahren nach einem der Ansprüche 1 bis 8, **dadurch gekennzeichnet**,
daß man synergetische Effekte durch Stoffkombinationen bei der letzten Nachbehandlung ausschließt und das verwendete Wasser von anderen Zusatzstoffen außer dem CO₂-Gas und der im Wasser gebildeten Kohlensäure und ggf. außer dem Duftstoff freihält.

10. Verfahren nach einem der Ansprüche 1 bis 9, **dadurch gekennzeichnet**,
daß man zu Überwachungszwecken den Zutrittsbereich des CO₂-Gases in das fließende Wasser mittels Schauglas oder dgl. von außen sichtbar gestaltet.

11. Verfahren nach einem der Ansprüche 1 bis 10, **dadurch gekennzeichnet**,
daß man den Nachbehandlungsvorgang intervallmäßig nach einem vorbestimmten Programm ausgestaltet, in dem der pH-Wert des der Druckwasserquelle entnommenen Wassers abwechselnd unverändert gelassen bzw. auf den vorbestimmten Wert abgesenkt wird, wobei als letzter Programmschritt eine Nachbehandlung mit durch CO₂-Gas abgesenktem pH-Wert durchgeführt wird.

12. Verfahren nach Anspruch 1 oder 2, **dadurch gekennzeichnet**,
(a) daß das Wasser mit variierendem Druck und variierender Strömungsgeschwindigkeit der Druckwasserquelle entnommen und über einen vorbestimmten Eintrittsquerschnitt (20) in eine Wasservorkammer (21) eines Druckmindergehäuses (3) eingeleitet und von dieser über einen gegenüber dem Eintrittsquerschnitt (20) deutlich verringerten Ausströmquerschnitt (22) in einen Ausströmkanal übergeleitet wird;
(b) daß der Auströmkanal über eine oder mehrere enge Verbindungsbohrungen (29) mit einem Druckgasraum (23) in ständig offener Verbindung gehalten wird, welcher mit einer Gasvorkammer (27) in dem Druckmindergehäuse verbunden wird;
(c) daß die Wasservorkammer (21) von der Gasvorkammer (27) durch eine flexible Membran getrennt wird und diese Membran (26) einerseits von dem Wassedruck in der Wasservorkammer (21) und andererseits von dem Gasdruck in der Gasvorkammer (27) beaufschlagt wird;
(d) daß die Gasvorkammer (27) über ein Schließventil (26a,25) mit einer CO₂-Druckgaspatrone (4) und die Membran (26) mit dem beweglichen Ventilglied (25) des Schließventils (26a,25) verbunden werden, derart, daß das Schließventil in Abhängigkeit vom Verhältnis des Wasserdrucks in der Wasservorkammer (21) und vom Gasdruck in der Gasvorkammer (27) im Öffnungssinne selbsttätig so gesteuert wird, daß unabhängig von den Schwankungen von Druck und Strömungsgeschwindigkeit des fließenden Wassers sein pH-Wert auf einem vorbestimmten abgesenkten Wert zwischen 5 und 6,7 konstant und genau gehalten wird.

13. Verfahren nach Anspruch 12, **dadurch gekennzeichnet**,
daß das Durchmesserverhältnis zwischen dem Einströmquerschnitt (20) und dem Ausströmquerschnitt (22) der Wasservorkammer (21) so eingestellt wird, daß sich der Wasserdruck im Ausströmkanal im Verhältnis zu dem Druck in der Wasservorkammer (21) auf einen Wert von etwa 1:2 oder niedriger selbsttätig einstellt.

14. Anordnung zum Ausführen des Verfahrens nach einem der Ansprüche 1 bis 3, mit einem Druckmindergehäuse (3) und einer Halterung für eine Druckgaspatrone (4), **dadurch gekennzeichnet**,
(a) daß das Druckmindergehäuse (3) eine Wasservorkammer (21) aufweist, die einen mit einer Versorgungsleitung verbindbaren Anschluß (20) von vorbestimmtem Eintrittsquerschnitt und einen mit der Entnahmestelle (5,6,7) verbundenen Ausströmkanal (22) von gegenüber dem Eintrittsquerschnitt deutlich verringertem Ausströmquerschnitt aufweist;
(b) daß der Ausströmkanal (22) über eine oder mehrere Verbindungsbohrungen (29) in ständig offener Strömungsverbindung mit einem Druckgasraum (23) steht, der mit einer Gasvorkammer (27) verbunden ist;
(c) daß die Gasvorkammer (27) mit der einen Seite und die Wasservorkammer (21) mit der anderen Seite einer flexiblen Membran (26) in freier Strömungsverbindung stehen;
(d) daß die Membran (26) über ein Betätigungsglied (26a) auf ein unter einer vorbestimmten Schließvorspannung stehendes Ventilglied (25) einwirkt, welches der Verbindung zwischen der Gasvorkammer (27) und der Halterung für die CO₂-Druckgaspatrone (4) zugeordnet ist, wobei die Druckgaspatrone selbsttätig abdichtend in die Aufnahme des Druckmindergehäuses (3) eingreift.

15. Kombination aus einer Druckgaspatrone (4) und einem Druckminderer (3), bestehend aus
(a) CO₂-Druckgaspatrone (4) mit einem zylindrischen Rumpf und einem Hals (Figur 3) zur Anbringung und zum Austausch an eine CO₂-Einströmöffnung des Druckminderer-Gehäuses (Gehäuseblock; 3) mit Wasser-Einström- und -Ausströmquerschnitten (20,22) zur Herstellung eines feinstimprägnierten Stoffgemisches aus Wasser und CO₂-Gas mit 400 mg CO₂ und mehr pro Liter Wasser und abgesenktem pH-Wert auf Werte deutlich unter 7 am Wasser-Ausströmquerschnitt (22);
(b) welche Druckgaspatrone (4) über ein Schließventil (26a,26,25) mit einer Gasvorkammer (27) im Druckminderergehäuse (3) verbindbar ist ;
(c) Membran (26), die mit einem beweglichen Schließglied (25) des Schließventiles (26a,26,25) über ein Betätigungsglied (26a) zusammenwirkt, um abhängig vom Wasserdruck in einer Wasservorkammer (21) des Druckminderergehäuses (3) und vom CO₂-Gasdruck aus der Druckgaspatrone (4) in der Gasvorkammer (27) das Schließventil (26a,26,25) im Öffnungssinne selbsttätig (automatisch) zu steuern, daß unabhängig von Druck- und Strömungsschwankungen des feinstimprägnierten Wassers am Ausströmquerschnitt (22) der vorbestimmte abgesenkte pH-Wert genau und zuverlässig aufrechterhalten wird.

16. Anordnung nach Anspruch 14 oder 15, **dadurch gekennzeichnet**,
daß die Membran/Ventilanordnung (25,26,26a) so einstellbar ist, daß der Gasdruck in der Gasvorkammer (27) zwischen dem Druck in der Wasservorkammer (21) und dem Druck im Bereich des Ausströmkanals (22) liegt.

17. Anordnung nach Ansprüchen 15 bis 16, **dadurch gekennzeichnet,**
daß das Durchmesserverhältnis zwischen dem Einströmquerschnitt und dem Ausströmquerschnitt für das Wasser so bemessen ist, daß der Druck in dem vom Wasser durchströmten Ausströmkanal (22) sich gegenüber dem Druck im Bereich des Einströmquerschnittes etwa wie 1:2 oder einem Mehrfachen davon verhält.

18. Anordnung nach einem der Ansprüche 14 bis 17, **dadurch gekennzeichnet**,
daß einer Verbindung (28) zwischen der Gasvorkammer (27) und dem Druckgasraum (23) eine einstellbare Nadeldüse zugeordnet ist.

19. Anordnung nach einem der Ansprüche 14 bis 18, **dadurch gekennzeichnet**,
daß das Durchmesserverhältnis von Einströmquerschnitt des fließenden Wassers und Querschnitt im Ausströmkanal in Abhängigkeit vom pH-Wert des zuströmenden Wassers einstellbar ist.

20. Anordnung nach einem der Ansprüche 14 bis 19, **dadurch gekennzeichnet**,
daß dem Einströmquerschnitt der Wasservorkammer (21) ein Durchfluß-Mengenbegrenzer zugeordnet ist.

21. Druckgaspatrone zur Verwendung bei einem Verfahren nach Anspruch 1 oder 2, **dadurch gekennzeichnet**,
daß sie CO₂ und einen Zusatzstoff enthält.

22. Druckgaspatrone nach Anspruch 21, **dadurch gekennzeichnet**,
daß der Zusatzstoff ein ätherischer Duftstoff oder ein ätherisches Öl ist.

23. Druckgaspatrone nach einem der Ansprüche 21 oder 22, **dadurch gekennzeichnet**,
daß sie CO₂ und den Zusatzstoff in einem solchen Mengenverhältnis enthält, daß bei der Entnahme das plötzliche Ausbleiben des Duftes die Entleerung der Patrone anzeigt.

24. Verwendung einer CO₂-Druckgaspatrone (4) mit einem zylindrischen Rumpf und einem demgegenüber eingezogenen Hals (Figur 3) zur Durchführung des Verfahrens gem. Anspruch 1 oder 2.

## Claims

1. A process for hair care, in which
(a) after a care treatment (washing, dyeing, perming treatments, hair conditioning pack step) the hair is subjected to a hair gloss rinsing step having acidic and astringent effect;
(b) for hair gloss rinsing the - optionally warm - water taken directly from a source of pressurized water (water supply line, flow heater, hot-water reservoir) is used, to which pure CO₂ gas as the only acid former pure CO₂ gas is added from a source of compressed CO₂ gas (4) with a pressure markedly below the static pressure of the water taken from the source of pressurized water at a point of the water flow path (22, 29) at which the static pressure of the water is lowered below the pressure of the CO₂ gas;
(c) the pH value being accurately and constantly adjusted to a certain value between 5 and 6, particularly 5.2.

2. The process for the production of a mixture of substances for the caring, cosmetic or therapeutic treatment of the skin, in which
as the only acid former pure CO₂ gas is supplied to the - optionally warm - water taken directly from a source of pressurized water (water supply line, flow heater, hot-water reservoir) from a source of compressed CO₂ gas (4) with a pressure markedly below the static pressure of the water taken from the source of pressurized water at a point of the water flow path (22, 29) at which the static pressure of the water is lowered below the pressure of the CO₂ gas, and the pH value is accurately and constantly adjusted to a predetermined value between about 5 and 6.7.

3. Use of - optionally warmed - water which is taken from a source of pressurized water and to which as the only acid former pure CO₂ gas is directly added from a source of compressed gas (4) in such a way that the pH value of the water adjusts constantly and accurately to a predetermined value between 6.7 and 5.2, at least for the last after-treatment of hair, skin of the head or skin of the body following a preceding care treatment.

4. The process according to any one of claims 1 to 3, characterized in that the water flowing at varying pressure from a source of pressurized water to a tapping point (7, 24) is used for automatically adjusting the pressure of the CO₂ taken from the source of compressed gas (4) to a pressure corresponding to the water pressure but being lower, whereupon the pressure of the running water drops to a value below the CO₂ gas pressure and the CO₂ and the water are contacted for direct free exchange.

5. The process according to any one of claims 1 to 4, characterized in that as a function of the quality, particularly the pH value, of the water taken from the source of pressurized water a predetermined fixed basic gas pressure value is preliminarily adjusted roughly, particularly by a single adjustment of an adjustable needle jet in the afflux (28) of the CO₂ gas.

6. The process according to any one of claims 1 to 5, characterized in that the CO₂ gas is contacted with an essential oil or an odorous substance before it is contacted with the running water.

7. The process according to claim 6, characterized in that the oil or the mixture consisting of oil and odorous substance is contacted dropwise with the CO₂ gas as early as in a compressed gas cylinder (4) - forming the source of compressed gas.

8. The process according to any one of claims 6 to 7, characterized in that during the operation the lacking perceptibility of the essential oil or odorous substance is simultaneously used as an indication of the depletion of a compressed gas cylinder (4) - forming the source of compressed gas.

9. The process according to any one of claims 1 to 8, characterized in that synergetical effects of combinations of substances are excluded in the last after-treatment and the water used is kept free from other additives, except the CO₂ gas and the carbonic acid formed in the water and optionally except the odorous substance.

10. The process according to any one of claims 1 to 9, characterized in that for the purpose of supervision the access area of the CO₂ gas into the running water is made visible from the outside by means of an inspection glass or the like.

11. The process according to any one of claims 1 to 10, characterized in that the after-treatment step is carried out at intervals according to a predetermined program in which the pH value of the water taken from the source of pressurized water is alternatingly left unchanged or lowered to the predetermined value, the last program step being an after-treatment with a pH value lowered by the CO₂ gas.

12. The process according to claim 1 or 2, characterized in
(a) that the water is taken from the source of pressurized water with varying pressure and varying flow rate and supplied to a water pre-chamber (21) of a pressure reducer housing (3) through a predetermined inlet cross-section (20), from where it is supplied to a discharge channel via an outlet cross-section (22) markedly reduced as compared to the inlet cross-section (20);
(b) that the discharge channel is kept in constant open communication with a compressed gas chamber (23) through one or more narrow connecting bores (29), which chamber is connected with a gas pre-chamber (27) in the pressure reducing housing;
(c) that the water pre-chamber (21) is separated from the gas pre-chamber (27) by a flexible membrane and this membrane (26) is subjected to the water pressure in the water pre-chamber (21), on the one hand, and the gas pressure in the gas-prechamber (27), on the other hand;
(d) that the gas pre-chamber (27) is connected with a compressed CO₂ gas cartridge (4) via a closing valve (26a, 25) and the membrane (26) is connected with the movable valve member (25) of the closing valve (26a, 25) such that, in the sense of opening, the closing valve is automatically controlled as a function of the relation of the water pressure in the water pre-chamber (21) and the gas pressure in the gas pre-chamber (27) in such a way that irrespective of the fluctuations of pressure and flow rate of the running water its pH value is kept constantly and accurately at a predetermined lowered value between 5 and 6.7.

13. The process according to claim 12, characterized in that the diameter ratio between inlet cross-section (20) and outlet cross-section (22) of the water pre-chamber (21) is adjusted in such a way that the water pressure in the discharge channel automatically adjusts at a value of about 1:2 or lower in relation to the pressure in the water pre-chamber (21).

14. A system for carrying out the process according to any one of claims 1 to 3, comprising a pressure reducing housing (3) and a support for a compressed gas cartridge (4), characterized in
(a) that the pressure reducing housing (3) includes a water pre-chamber (21) having a supply (20), connectable with a supply line, of predetermined inlet cross-section and a discharge channel (22), connected with the tapping point (5, 6, 7), of an outlet cross-section markedly reduced as compared to the inlet cross-section;
(b) that the discharge channel (22) is in constant open flow communication with a compressed gas chamber (23) through one or more connecting bores (29), which chamber is connected with a gas pre-chamber (27);
(c) that the gas pre-chamber (27) is in free flow communication with one side of a flexible membrane (26) and the water pre-chamber (21) is in free flow communication with the other side thereof;
(d) that via an actuation member (26a) the membrane (26) acts upon a valve member (25) which is under a predetermined closing pretension and assigned to the connection between gas pre-chamber (27) and support for the compressed CO₂ gas cartridge (4), the compressed gas cartridge engaging the receiver of the pressure reducing housing (3) in an automatically sealing manner.

15. A combination consisting of a compressed gas cartridge (4) and a pressure reducer (3), consisting of
(a) a compressed CO₂ gas cartridge (4) having a cylindrical body and a neck (Fig. 3) for the attachment and for the exchange to a CO₂ inlet opening of the pressure reducer housing (housing block; 3) having water inlet and outlet cross-sections (20, 22) for the production of a most finely impregnated mixture of substances consisting of water and CO₂ gas with 400 mg CO₂ and more per liter of water and a pH value lowered to values markedly below 7 at the water outlet cross-section (22);
(b) which compressed gas cartridge (4) can be connected through a closing valve (26a, 26, 25) with a gas pre-chamber (27) in the pressure reducer housing (3);
(c) membrane (26) which cooperates with a movable closing member (25) of the closing valve (26a, 26, 25) via an actuation member (26a) to spontaneously (automatically) control the closing valve (26a, 26, 25) in the sense of opening as a function of the water pressure in a water pre-chamber (21) of the pressure reducer housing (3) and of the CO₂ gas pressure from the compressed gas cartridge (4) in the gas pre-chamber (27), that the predetermined lowered pH value is maintained accurately and reliably at the outlet cross-section (22) irrespective of pressure and flow fluctuations of the most finely impregnated water.

16. The system according to claim 14 or 15, characterized in that the membrane/valve means (25, 26, 26a) is adjustable in such a way that the gas pressure in the gas pre-chamber (27) is between the pressure in the water pre-chamber (21) and the pressure in the area of the discharge channel (22).

17. The system according to claims 15 to 16, characterized in that the diameter ratio between the inlet cross-section and the outlet cross-section for the water is such that the pressure in the discharge channel (22) through which the water flows in relation to the pressure in the area of the inlet cross-section is about 1:2 or a multiple thereof.

18. The system according to any one of claims 14 to 17, characterized in that an adjustable needle jet is assigned to a connection (28) between the gas pre-chamber (27) and the compressed gas chamber (23).

19. The system according to any one of claims 14 to 18, characterized in that the diameter ratio of the inlet cross-section of the running water and the cross-section in the discharge channel is adjustable as a function of the pH value of the affluent water.

20. The system according to any one of claims 14 to 19, characterized in that a flow regulator is assigned to the inlet cross-section of the water pre-chamber (21).

21. A compressed gas cartridge for use in a process according to claim 1 or 2, characterized in that it contains CO₂ and an additive.

22. The compressed gas cartridge according to claim 21, characterized in that the additive is an essential odorous substance or an essential oil.

23. The compressed gas cartridge according to any one of claims 21 or 22, characterized in that it contains CO₂ and the additive in such a quantity ratio that during the withdrawal the sudden absence of the odor indicates the depletion of the cartridge.

24. Use of a compressed CO₂ gas cartridge (4) having a cylindrical body and a neck recessed as compared thereto (Fig. 3) for carrying out the process according to claim 1 or 2.

## Revendications

1. Procédé destiné au soin de la chevelure dans lequel
(a) suite à un traitement de soin (lavage, teinture, mise en forme, soin nutritionnel), les cheveux sont soumis à un rinçage de brillance de la chevelure à effets acidifiant et adstringent;
(b) le rinçage de brillance de la chevelure est fait à l'aide d'eau - éventuellement chaude - prélevée directement sur une source d'eau comprimée (conduite d'alimentation en eau, chauffe-eau instantané, ballon d'eau chaude), d'eau à laquelle est additionné comme acidificateur unique du gaz CO₂ pur provenant d'une source de gaz comprimé (4) présentant une pression nettement inférieure à la pression statique de l'eau prélevée sur la source d'eau comprimée, et cela à un endroit de la voie d'écoulement (22,29) de l'eau où la pression statique de l'eau est ramenée au-dessous de la pression que présente le gaz CO₂;
(c) la valeur pH étant réglée de manière précise et constante à une valeur déterminée entre 5 et 6, en particulier de 5,2.

2. Procédé de fabrication d'un mélange de substances destiné au traitement soignant, cosmétique ou thérapeutique de la peau dans lequel l'eau - éventuellement chaude - prélevée directement sur une source d'eau comprimée (conduite d'alimentation en eau, chauffe-eau instantané, ballon d'eau chaude) est alimentée en gaz CO₂ comme acidificateur unique provenant d'une source de gaz comprimé (4) présentant une pression qui est nettement inférieure à la pression statique de l'eau prélevée sur la source d'eau comprimée à un endroit de la voie d'écoulement (22,29) de l'eau où la pression statique de l'eau est réduite au-dessous de la pression du gaz CO₂, la valeur pH étant réglée de manière précise et constante à une valeur prédéfinie entre environ 5 et 6,7.

3. Utilisation d'eau - éventuellement réchauffée - prélevée sur une source d'eau comprimée et étant directement mélangée à du gaz CO₂ provenant d'une source de gaz comprimé (4) comme acidificateur unique de manière que la valeur pH de l'eau se règle de manière précise et constante à une valeur prédéterminée entre 6,7 et 5,2, pour au moins le dernier traitement de la chevelure, du cuir chevelu ou de la peau suite à un après-shampooing précédent.

4. Procédé suivant l'une des revendications 1 à 3, **caractérisé en ce que** l'eau prélevée sur la source d'eau comprimée coulant sous des pressions variables vers une prise d'eau (7,24) est utilisée pour le réglage automatique de la pression du CO₂ prélevé sur la source de gaz comprimé (4) à une valeur correspondant à celle de la pression d'eau, toutefois inférieure à celle-ci, la pression de l'eau courante tombant par la suite à une valeur inférieure à celle de la pression du gaz CO₂ et le CO₂ étant mis en libre échange direct avec l'eau.

5. Procédé suivant l'une des revendications 1 à 4, **caractérisé en ce que** l'on prérègle de manière grossière une valeur de base de la pression du gaz fixe prédéterminée en fonction de la qualité, en particulier de la valeur pH de l'eau prélevée sur la source d'eau comprimée, notamment en procédant à un ajustage unique d'une buse à aiguille sur la voie d'amenée (28) du gaz CO₂.

6. Procédé suivant l'une des revendications 1 à 5, **caractérisé en ce que** l'on met le gaz CO₂, avant qu'il soit mis en contact avec l'eau courante, en contact avec une huile volatile ou une substance odorante.

7. Procédé suivant la revendication 6, **caractérisé en ce que** l'on met en contact goutte à goutte l'huile ou le mélange d'huile et de la substance odorante avec le gaz CO₂ déjà dans un cylindre à gaz comprimé (4).

8. Procédé suivant l'une des revendications 6 à 7**, caractérisé en ce qu'**en service, on prend comme indicateur de l'épuisement d'un cylindre à gaz comprimé (4) - constituant la source de gaz comprimé - l'arrêt de la perceptibilité de l'huile volatile ou de la substance odorante.

9. Procédé suivant l'une des revendications 1 à 8, **caractérisé en ce que** lors du dernier après-shampooing on exclut des effets synergiques dus à des combinaisons de substances et que l'on exempte l'eau utilisée d'autres substances, expression faite du gaz CO₂ ainsi que du dioxyde de carbone s'étant formé dans l'eau et, le cas échéant, de la substance odorante.

10. Procédé suivant l'une des revendications 1 à 9, **caractérisé en ce que** pour des fins de contrôle, la zone d'entrée du gaz CO₂ dans l'eau courante est rendue transparente depuis l'extérieur à l'aide d'un voyant ou semblable.

11. Procédé suivant l'une des revendications 1 à 10, **caractérisé en ce que** le processus d'après-shampooing se déroule en intervalles suivant un programme prédéterminé prévoyant alternativement le maintien respectivement la réduction de la valeur pH de l'eau prélevée sur la source d'eau comprimée à la valeur prédéfinie, un après-shampooing à valeur pH réduite grâce au gaz CO₂ étant réalisé en tant que dernière étape du programme.

12. Procédé suivant la revendication 1 ou 2, **caractérisé en ce que**
(a) l'eau est prélevée à pression et vitesse d'écoulement variables sur la source d'eau comprimée et introduite par une section d'entrée (20) prédéfinie dans une préchambre d'eau (21) d'une boîte de détente (3) et amenée depuis celle-ci dans un canal de sortie, en passant par une section de sortie (22) visiblement réduite par rapport à la section d'entrée (20) ;
(b) le canal de sortie, grâce à un ou plusieurs alésages de liaison (29), est maintenu en permanence en liaison ouverte avec un espace de gaz comprimé (23) raccordé à une préchambre de gaz (27) prévue dans la boîte de détente;
(c) la préchambre d'eau (21) est séparée de la préchambre de gaz (27) à l'aide d'une membrane flexible et sur ladite membrane (26) est d'une part appliqué la pression d'eau régnant dans la préchambre d'eau (21) et d'autre part la pression du gaz rencontrée dans la préchambre de gaz (27);
(d) la préchambre de gaz (27) est raccordée, par l'intermédiaire d'une vanne de fermeture (26a,25), à une cartouche à gas comprimé CO₂ (4) et la membrane (26) est raccordée au membre flexible (25) de la vanne de fermeture (26a,25), de manière que la vanne de fermeture soit automatiquement commandée en fonction du rapport entre la pression d'eau régnant dans la préchambre d'eau (21) et la pression du gaz régnant dans la préchambre de gaz (27) dans le sens de l'ouverture que quelles que soient les variations de pression et de vitesse d'écoulement de l'eau courante, la valeur pH de celle-ci soit maintenue de façon constante et précise à une valeur prédéterminée réduite entre 5 et 6,7.

13. Procédé suivant la revendication 12, **caractérisé en ce que** le rapport de diamètre entre la section d'entrée (20) et la section de sortie (22) de la préchambre d'eau (21) est réglé de manière que la pression d'eau s'ajuste automatiquement en rapport à la pression régnant dans la préchambre d'eau (21) à une valeur de 1:2 ou inférieure.

14. Disposition pour la mise en oeuvre du procédé suivant l'une des revendications 1 à 3, comprenant une boîte de détente (3) et un support d'une cartouche à gaz comprimé (4), **caractérisé en ce que**
(a) la boîte de détente (3) comprend une préchambre d'eau (21) munie d'un raccordement (20) de section d'entrée prédéfinie susceptible d'être raccordé à une conduite d'alimentation ainsi qu'un canal de sortie (22) raccordé à la prise (5,6,7) de section de sortie nettement réduite par rapport à la section d'entrée;
(b) le canal de sortie (22), grâce à un ou plusieurs alésages de liaison (29), est maintenu en permanence en liaison d'écoulement ouverte avec un espace de gaz comprimé (23) raccordé à une préchambre de gaz (27);
(c) la préchambre de gaz (27) est en liaison d'écoulement libre avec l'une des deux faces et la préchambre d'eau (21) avec l'autre face de la membrane flexible (26);
(d) à l'aide d'un membre d'actionnement (26a), la membrane (26) agit sur un membre de vanne (25) présentant une précontrainte de fermeture prédéterminée et étant attribué à la liaison entre la préchambre de gaz (27) et le support de la cartouche à gaz comprimé CO₂ (4), la cartouche à gaz comprimé prenant de manière auto-étanchéifiante dans le logement de la boîte de détente (3).

15. Combinaison d'une cartouche à gaz comprime (4) et d'un détendeur (3) comprenant
(a) la cartouche à gaz comprimé (4) présentant un corps cylindrique et un col (Fig. 3) pour être montée et échangée sur une ouverture d'entrée de CO₂ faisant partie de la boîte de détente (bloc de boîte; 3), comprenant des sections d'entrée et de sortie d'eau (20,22) pour la préparation d'un mélange de substances super-imprégné d'eau et de gaz CO₂ à 400 mg de CO₂ et plus par litre d'eau et à valeur pH réduite à des valeurs nettement inférieures à 7 à la section de sortie de l'eau (22);
(b) ladite cartouche à gaz comprimé (4) étant susceptible d'être raccordée, par l'intermédiaire d'une vanne de fermeture (26a,26,25), à une préchambre de gaz (27) prévue dans la boîte de détente (3);
(c) une membrane (26) coopérant avec un membre de fermeture flexible (25) de la vanne de fermeture (26a,26,25), par l'intermédiaire d'un membre d'actionnement (26a), afin de commander (automatiquement), dans la préchambre de gaz (27), la vanne de fermeture (26a,26,25) dans le sens de l'ouverture, en fonction de la pression d'eau régnant dans une préchambre d'eau (21) de la boîte de détente (3) et de la pression du gaz CO₂ provenant de la cartouche à gaz comprimé (4), de manière que la valeur pH réduite prédéterminée soit maintenue de manière précise et sûre, quelles que soient les variations de pression et d'écoulement de l'eau super-imprégnée à la section de sortie (22).

16. Disposition suivant l'une des revendications 14 ou 15, **caractérisé en ce que** l'agencement membrane/vanne (25,26,26a) est ajustable de manière que la pression du gaz régnant dans la préchambre de gaz (27) soit comprise entre la pression régnant dans la préchambre d'eau (21) et celle rencontrée au droit du canal de sortie (22).

17. Disposition suivant les revendications 15 à 16, **caractérisé en ce que** le rapport de diamètre entre la section d'entrée et la section de sortie de l'eau est dimensionné de manière que la pression rencontrée dans le canal de sortie (22) traversé par l'eau soit d'environ 1:2 ou équimultiple vis-à-vis de la pression régnant au droit de la section de sortie.

18. Disposition suivant l'une des revendications 14 à 17, **caractérisé en ce qu'**une buse à aiguille ajustable est attribuée à une liaison (28) entre la préchambre de gaz (27) et l'espace de gaz comprimé (23).

19. Disposition suivant l'une des revendications 14 à 18, **caractérisé en ce que** le rapport de diamètre entre la section d'entrée de l'eau courante et la section du canal de sortie est réglable en fonction de la valeur pH de l'eau affluente.

20. Disposition suivant l'une des revendications 14 à 19, **caractérisé en ce qu'**à la section d'entrée de la préchambre d'eau (21) est attribué un limiteur de débit.

21. Cartouche à gaz comprimé destinée à être utilisée dans un procédé suivant la revendication 1 ou 2, **caractérisé en ce qu'**elle contient du CO₂ et un adjuvant.

22. Cartouche à gaz comprimé suivant la revendication 21, **caractérisé en ce que** l'adjuvant est soit une substance odorante volatile soit une huile volatile.

23. Cartouche à gaz comprimé suivant l'une des revendications 21 ou 22, **caractérisé en ce qu'**elle contient du CO₂ et l'adjuvant dans un rapport de quantité tel que lors de l'extraction, le manque soudain de l'odeur indique l'épuisement de la cartouche.

24. Utilisation d'une cartouche à gaz comprimé (4) ayant un corps cylindrique et un col rentré par rapport à celui-ci (Fig. 3) pour la mise en oeuvre du procédé suivant la revendication 1 ou 2.
